(19) Europäisches Patentamt
European Patent Office
Office européen des brevets

(11) Publication number: **0 351 020**
**A2**

(12) # EUROPEAN PATENT APPLICATION

(21) Application number: **89201842.5**

(51) Int. Cl.[4]: **A01N 43/08 , C07D 307/60**

(22) Date of filing: **11.07.89**

(30) Priority: **13.07.88 GB 8816615**

(43) Date of publication of application:
**17.01.90 Bulletin 90/03**

(84) Designated Contracting States:
**BE CH DE ES FR GB GR IT LI NL SE**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Raven, Clive Alan**
**Hemsby House Hearts Delight**
**Borden Nr. Sittingbourne Kent(GB)**
Inventor: **Jenkins, Elizabeth Sarah**
**Southbourne 28 Lingfield Road**
**Gravesend Kent(GB)**
Inventor: **Lathbury, David Charles**
**17 Diligent Drive**
**Sittingbourne Kent(GB)**

(74) Representative: **Bennett, David Arthur Horder et al**
**4, York Road**
**London SE1 7NA(GB)**

(54) **Substituted heterocyclic herbicides.**

(57) A method of combating undesired plant growth comprises the pre-emergent application to soil of 3-(1-amino)-ethylidene-5-methyl-furan-2,4-dione.

EP 0 351 020 A2

## SUBSTITUTED HETEROCYCLIC HERBICIDES

This invention relates to the use of certain substituted furandiones in combating undesired plant growth. The invention also relates to certain novel furandiones.

The effective control of perennial weeds has long posed a problem in agriculture. Commercially available herbicides (such as 2,4-dichlorophenoxy-acetic acid and 2-amino-1,3,4-triazole) which are capable of acting to control perennial weeds are conventionally applied directly onto the weeds as foliar sprays in order to realise their maximum effectiveness. Such herbicides thus only effectively control those weeds which have emerged at the time of spraying. There is a need for the provision of means to control such weeds as they emerge.

It has been recognised for a considerable length of time that certain compounds having an unsaturated lactone ring such as coumarins and tetronic acids are capable of causing chlorophyll inhibition in plants. Thus Hamner and Tukey, Botannical Gazette, 1951, pages 525-528 reported that the compound 3-(alpha-imino- ethyl)-5-methyl tetronic acid, when used to soak seeds or spray seedlings and plants, caused chlorophyll inhibition in wheat, cucumber, sweet corn, radish and red kidney bean. Although the authors remarked that the tetronic acid may have value as a herbicide and that the compound appears to be extremely effective on monocotyledonous plants, they did not postulate any mechanism for the observed effects. Chlorophyll inhibition is known to be caused by many different agents including antibiotics, plant hormones, amino acid analogues, herbicides and growth retardants by a variety of mechanisms - see the review article by Wolfe (The Botanical Review, Vol. 43, No 4, 1977, pages 395 to 425).

It has surprisingly now been found that the furandione, 3-(1 amino)ethylidene-5-methyl-furan-2,4-dione, has a spectrum of activity which renders the compound particularly suitable for pre-emergence soil treatment for the control of weeds especially perennial weeds (including broadleaved perennial weeds), having an undergound perennating structure.

Accordingly, the present invention provides a method of combating undesired plant growth in soil which comprises applying to the soil prior to the emergence of at least some of the undesired plant growth, 3-(1-amino)ethylidene-5-methyl-furan-2,-4-dione.

The compound 3-(1-amino)ethylidene-5-methyl-furan-2,4-dione, which is known, may be obtained, for example, by the method of Tanaka et al (Chem. Pharm. Bull., Vol 27, No 8, 1979, pages 1901-1906).

The enantiomers of 3-(1-amino)ethylidene-5-methyl-furan-2,4-dione are novel and useful as herbicides and therefore the invention also includes as a further aspect thereof the S- and R- enantiomers of the compound of formula I

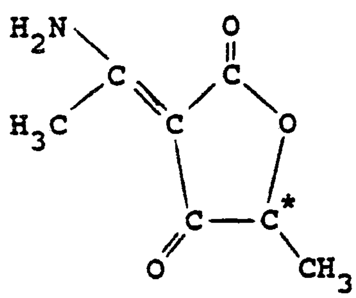

(I)

where C* represents an optically active carbon atom.

It will, of course, be appreciated that the S-and R- enantiomers of the compound of formula I will exist in cis or trans form or as mixtures of cis and trans isomers.

The enantiomers of the compound of formula I may be prepared from the corresponding optically active 3-acetyl derivatives of formula II

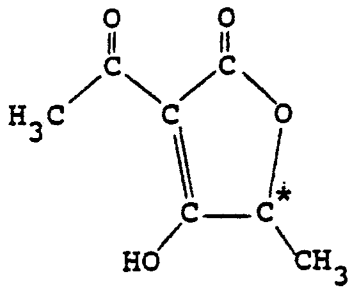

(II)

by reaction of the compound of formula II with ammonia, suitably in an organic solvent such as ethanol, preferably at elevated temperature.

The optically active acetyl derivatives of formula II may be derived from the commercially available optically active lactic acid esters, such as methyl (R)-lactate and methyl (S)-lactate, by reaction of the lactate with diketene to give ethyl $\alpha$ -(acetoacetoxy)propionate which is then cyclised for example in the presence of an alkali metal oxide such as potassium t-butoxide, to yield an optically active compound of formula II. This procedure is described by Boll et al, Acta Chemica Scandinavia, Vol. 22, 1968, pages 3251-3255.

The invention also includes herbicidal compositions comprising an enantiomer of formula I in association with at least one carrier and a method of making such a composition which comprises bringing an enantiomer of formula I into association with at least one carrier.

Conveniently, the furan-2,4-dione is applied in herbicidal composition form as active ingredient together with a suitable carrier. Other herbicidally active ingredients may additionally be employed in such a composition. Examples of such herbicides are given in our copending Application No. (T 610).

The furandione may be applied to the soil before the planting or sowing of a crop therein. Alternatively the compound may be applied to the soil after sowing or planting of a crop, or even after harvesting of a crop in order to clear the soil of undesired plant growth prior to the further use of the land.

According to one preferred aspect of the invention, the furandione is applied to the soil before or after the sowing of oil seed rape.

The dosage of active ingredient used may, for example, be in the range of from 0.05 to 10kg/ha preferably in the range of from 1 to 5 kg/ha.

A carrier used in a herbicidal composition comprising the furandione is any material with which the active ingredient is formulated to facilitate application to the locus to be treated or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montomorillonites and micas; calcium carbonate, calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition used in accordance with the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid

esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions used in the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing $\frac{1}{2}$-10% w of active ingredient. Granules are usually prepared to have a size between 10 and 100 BS mesh (1.676 - 0.152 mm), and may be manufactured by agglomeration or impregnation techniques. Generally, granules will contain $\frac{1}{2}$-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The compositions used in the invention may also contain other ingredients, for example other compounds possessing insecticidal or fungicidal properties, as well as other herbicidally active ingredients such as those described in our copending Application No.      (T 610).

The invention will now be described with reference to the following examples.

## Example 1

### Preparation of (S)-3-(1-amino)ethylidene-5-methyl-furan-2,4-dione

#### a) Methyl (S)-α-(acetoacetoxy)-propionate

To a mixture of (S)-methyl lactate (14g, 0.134 moles) and triethylamine (0.2g, 1.9 mmoles), was added diketene at 65°-75° C dropwise over 40 minutes with rapid stirring. The reaction was stirred at 70° C for a further 30 minutes, cooled to room temperature and distilled at reduced pressure to give the title compound as a colourless oil. B.p. 96°-98° C 0.1 mmHg. Yield 19.1gm (85%).

#### b) (S)-3-acetyl-5-methyl-furan-2,4-dione

To a solution of the ester prepared in part a) above (8g, 46.5mmoles) in dry t-butanol (17ml) was added potassium t-butoxide (4.8gm, 43 mmoles) in one portion with rapid stirring. The reaction was stirred at reflux under nitrogen for 2 hours, cooled to room temperature, acidified with 6N HCl and extracted with chloroform (3x20ml). The combined extracts were dried ($MgSO_4$) and solvent removed.

The crude product was redissolved in ether (20ml) and cooled to -78°C for 20 minutes during which time the product recrystallised. The product was filtered and further purified by a second low temperature recrystallisation to give the title compound as a white solid, mp. 55°-56°C. Yield 4.3g (65%). [α ]=-38.9 C=0.0095 in ethanol.

c) (S)-3-(1-amino)ethylidene-5-methyl-furan-2,4-dione

To a solution of the compound prepared in part (b) above (0.3g, 1.9 mmol) in absolute ethanol (1ml) was added a solution of ammonia (10% w/v) in ethanol (4 ml). The mixture was heated in a sealed tube for 2 hours at 100°C, cooled to room temperature and the solvent removed. The product was purified by chromatography on silica, eluting with ethyl acetate to give the title compound as a white solid, mp. 157-159°C. Yield 0.29g (96%). [α]=-45.51 C=0,0145 in ethanol.

Example 2

Pre-Emergent Herbicidal Activity

To evaluate herbicidal activity, the compound (S)-3-(1-amino)ethylidene-5-methyl-furan-2,4-dione ("dione") and the commercially available compound 3-amino-1H-1,2,4-triazole ("ATA") were tested using the representative range of plant species given in Table 1.

Table 1

| Species | | Species Code |
|---|---|---|
| Wheat cv.Sicco | (*Triticum aestivum*) | WH |
| Maize cv.Beaupre pau 205 | (*Zea mays*) | MA |
| Vine cv. Cab sauv | (*Vitis vinifera*) | VN |
| Couchgrass | (*Elymus repens*) | CO |
| Johnson grass | (*Sorghum halepense*) | JG |
| Bindweed (Hedge)regrowth | (*Calystegia sepium*) | SE |
| Bindweed (Field) | *Convolvulus arvensis*) | CV |

Vine was treated as cuttings at bud break. Wheat and maize were sown as seeds and the remaining species were propagated from perennating organs in semi-sterilised loam in 12.5 cm pots.

The tests involved spraying a liquid formulation of the compound onto the soil to which the species in Table 1 had been introduced. The dione was formulated in a 1:1 acetone:water mixture containing up to 0.2% of an alkylphenyl/ethylene oxide condensate available under the trade mark TRITON X-155. ATA was formulated by mixing with tap water a mixture of 3-amino-1H-1,2,4-triazole and ammonium thiocyanate available under the trade mark WEEDAZOL TL. Test solutions were applied as single dose pre emergence sprays in a total volume of 600 l/ha using the dosage levels given in Table 2 below. Maintenance was by sub-irrigation via capillary matting with supplementary overhead watering as required.

The herbicidal effects of the test compounds were assessed visually 39 days after treatment and recorded on a standard 0-9 scale where rating 0 indicates growth as untreated control and rating 9 indicates death. An increase of 1 unit on the linear scale approximates to a 10% increase in the level of effect.

The results of the tests, converted to percentages, are set out in Table 2 below. From the Table it can be seen that after 39 days the furandione has a significantly higher percentage herbicidal effect when applied to the soil pre-emergence than has the commercial herbicide, ATA.

TABLE 2

| Compound | Dose kg/ha | WH 39 Days | MA 39 Days | VN 39 Days | CO 39 Days | JG 39 Days | SE 39 Days | CV 39 Days | Mean |
|---|---|---|---|---|---|---|---|---|---|
| DIONE | 4.8 | 72.22 | 93.06 | 80.56 | 94.44 | 88.89 | 100.00 | 97.22 | 89.48 |
| DIONE | 2.4 | 51.39 | 84.72 | 77.78 | 91.67 | 76.39 | 95.83 | 91.67 | 81.35 |
| DIONE | 1.2 | 20.83 | 81.94 | 73.61 | 84.72 | 61.11 | 94.44 | 75.00 | 70.24 |
| ATA | 9.6 | 16.67 | 5.56 | 19.44 | 44.44 | 20.83 | 36.11 | 30.56 | 24.80 |
| ATA | 4.8 | 0.00 | 0.00 | 15.28 | 19.44 | 20.83 | 8.33 | 13.89 | 11.11 |
| ATA | 2.4 | 0.00 | 0.00 | 0.00 | 6.94 | 12.50 | 0.00 | 12.50 | 4.56 |
| CONTROL | - | 0.00 | 0.00 | 0.00 | 0.00 | 0.00 | 0.00 | 0.00 | 0.00 |

Example 3

Herbicidal Activity in Oil Seed Rape

To evaluate pre-emergence selectivity in oil seed rape, the compound (S)-3-(1-amino) ethylidene-5-methyl-furan-2,4-dione ("dione") was compared with the commercial available pre-emergence herbicides 2-chloro-4,6-bis (ethylamino)-1,3,5-triazine (known as "simazine") and 2,6-dichlorobenzonitrile (known as "dichlobenil").

The tests were carried out as described in Example 2.

The formulations used were technical materials in acetone: water containing TRITON X-155 as described in Example 2 for the dione.

The plant species treated were wheat, couchgrass and hedge bindweed as given in Table 1, together with oil seed rape (Brassica napus) RA. Wheat, couchgrass and bindweed are important weeds affecting oil seed rape crops.

The herbicidal effects of the test compounds were assessed visually after 28 days. The results of the tests, on a visual 0-9 scale and converted to percentage effect results as described in Example 2, are set out in Table 3 below. From the Table it can be seen that,unlike either of the two commercial herbicides tested,the furandione generally combines a good herbicidal activity against the weed species, especially the broadleaved species SE, with a low activity against the oil seed rape crop species RA.

TABLE 3

| Treatment | Dose kg/ha | WH 28 dat | RA 28 dat | CO 28 dat | SE 28 dat |
|---|---|---|---|---|---|
| Dione | 6.0 | 100.00 | 22.22 | 100.00 | 100.00 |
| Dione | 2.0 | 90.74 | 0.00 | 94.44 | 100.00 |
| Dione | 0.6 | 24.07 | 0.00 | 81.48 | 100.00 |
| Dione | 0.2 | 9.26 | 0.00 | 51.85 | 77.78 |
| Simazine | 6.0 | 100.00 | 92.59 | 92.59 | 100.00 |
| Simazine | 2.0 | 96.30 | 74.07 | 70.37 | 96.30 |
| Simazine | 0.6 | 14.81 | 62.96 | 51.85 | 84.19 |
| Simazine | 0.2 | 0.00 | 0.00 | 18.52 | 0.00 |
| Dichlobenil | 6.0 | 100.00 | 100.00 | 98.15 | 100.00 |
| Dichlobenil | 2.0 | 81.48 | 85.19 | 22.22 | 77.18 |
| Dichlobenil | 0.6 | 11.11 | 0.00 | 3.70 | 0.00 |
| Dichlobenil | 0.2 | 0.00 | 0.00 | 0.00 | 0.00 |

**Claims**

1. A method of combating undesired plant growth in soil which comprises applying to the soil prior to the emergence of at least some of the undesired plant growth, 3-(1-amino)ethylidene-5-methyl- furan-2,4-dione.

2. A method as claimed in claim 1, wherein racemic 3-(1-amino)ethylidene-5-methyl-furan-2,4-dione is applied to the soil.

3. A method according to claim 1, wherein an S enantiomer of 3-(1-amino)ethylidene-5-methyl-furan-2,4-dione is applied to the soil.

4. A method as claimed in any of claims 1 to 3, wherein the furandione is applied to the soil before the planting or sowing of a crop therein.

5. A method as claimed in any one of claims 1 to 3, wherein the furandione is applied to the soil after sowing or planting of a crop therein.

6. A method as claimed in any one of claims 1 to 5, wherein the furandione is applied to soil in which oil seed rape has been sown or is to be sown.

7. A method as claimed in any one of claims 1 to 6, wherein the furandione is applied to the soil in association with a carrier.

8. A method as claimed in any one of claims 1 to 7, wherein the furandione is applied to the soil in an amount in the range of from 1 to 5kg/ha.

9. An S- or R-enantiomer of the compound of formula I

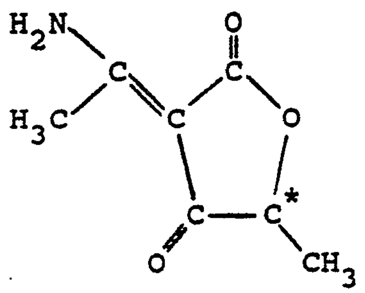

(I)

where C* represents an optically active carbon atom.

10. A process for the preparation of an enantiomer as claimed in claim 9, which comprises reacting a compound of formula II

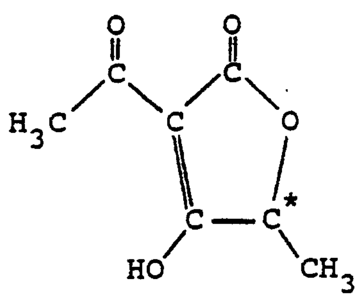

(II)

where C* represents an optically active carbon atom, with ammonia.

11. A compound as defined in claim 9 whenever prepared by a process as claimed in claim 10.

12. A herbicidal composition which comprises a compound as claimed in claim 9 or claim 11, together with a carrier.

13. A composition as claimed in claim 12, which comprises at least two carriers, at least one of which is a surface active agent.

14. The use of a compound as claimed in claim 9 or claim 11, as a herbicide.